Europäisches Patentamt

European Patent Office

Office européen des brevets

(19)

(11) Publication number: **0 307 241 B1**

# EUROPEAN PATENT SPECIFICATION

(12)

(45) Date of publication of patent specification: **23.12.92**  (51) Int. Cl.⁵: **A61B  17/58**, A61F 2/44

(21) Application number: **88308375.0**

(22) Date of filing: **09.09.88**

(54) Surgical prosthetic implant.

(30) Priority: **11.09.87 US 95461**
**28.03.88 US 173928**

(43) Date of publication of application:
**15.03.89 Bulletin  89/11**

(45) Publication of the grant of the patent:
**23.12.92 Bulletin  92/52**

(84) Designated Contracting States:
**AT CH DE FR GB LI SE**

(56) References cited:
**EP-A- 0 042 271**
**DE-A- 3 505 567**
**US-A- 4 501 269**

(73) Proprietor: **Brantigan, John W.**
**328 Overlook Brook Court**
**Chagrin Falls Ohio 44022(US)**

(72) Inventor: **Brantigan, John W.**
**328 Overlook Brook Court**
**Chagrin Falls Ohio 44022(US)**

(74) Representative: **McCall, John Douglas et al**
**W.P. THOMPSON & CO. Coopers Building**
**Church Street**
**Liverpool L1 3AB(GB)**

Rank Xerox (UK) Business Services

## Description

This invention relates to the art of prosthetic devices adapted to be inserted transversely in a vertebral column on prepared sites of the opposed faces of adjacent vertebrae forming struts which are fused into the vertebrae to maintain a normal disc space between the vertebrae. These devices are, when inserted in the vertebral column, in the form of rigid inert plugs spanning the disc space in side-by-side relation and having roughened surfaces facilitating ingrowth of bone tissue.

The plugs are mounted endwise on a tool to facilitate insertion between the adjacent vertebrae, have a height that will reclaim the normal disc space and can stretch remaining tissue of a collapsed damaged disc. Preferred plugs have barbs biting into the vertebrae, slots for carrying bone graft material, tapered leading ends facilitating insertion between the vertebrae and are formed from a radiolucent material.

The leading cause of low back pain arises from rupture or degeneration of lumbar intervertebral discs. Pain in the lower extremeties of the back (sciatica) is caused by compression of spinal nerve roots by damaged discs between the vertebrae and low back pain is caused by collapse of the disc and the adverse effects of bearing the majority of the body weight through a damaged unstable vertebral joint. Surgical treatments for relief of the sciatic pain and lower back pain generally include the following:

### 1.) Excision Of The Ruptured Soft Disc

This procedure removes the portion of the disc compressing the spinal nerve and is generally successful in relieving the sciatic leg pain but in more than half of the cases, there is a recurrence of back pain. Over a period of time the disc gradually loses height due to the rupture and this loss of height causes the posterior facet joints of the vertebrae to fit incorrectly resulting in arthritic change in all elements of the spinal segment. Recurrent nerve root compression due to bony encroachment (spinal stenosis) also develops. The continuing and recurring back pain from this source has created a leading source of pain and disability.

### 2.) Disc Excision With Posterior Fusion

Traditional posterior fusion, creating bone growth between the bony laminae, or postero-lateral fusion between the transverse processes prevents motion between the adjacent vertebrae but does not alter the fact that approximately 90% of the body weight must be transmitted through degenerated discs causing pain. Further, posterior fusion tends to cause bony overgrowth leading to nerve root compression by spinal stenosis.

### 3.) Disc Excision With Anterior Interbody Fusion

Interbody fusion techniques, in which the soft disc is completely excised and replaced with either the patient's own bone (autologous bone) or with transplant banked bone (homologous bone) are generally successful if solid fusion can be obtained between adjacent vertebrae bodies. Unfortunately, the success rate has only been about 50%.

### 4.) Disc Excision With Posterior Lumbar Intervertebral Fusion (PLIF)

This procedure reconstructs the normal anatomic relationships between the bony and the neural structures and has many advantages. Weight bearing through a solid bony fusion made between vertebral bodies relieves the mechanical pain of the traditional unstable degenerative disc and generally prevents long term disc collapse or further degenerative changes. The complete disc excision prevents recurrent herniation of the same degenerated disc.

However, this PLIF procedure has several serious disadvantages in that it is technically very difficult, and, therefore, not as successful or widely used as it might be. It entails large amounts of blood loss in a small deep hole causing physiological stress to the patient and psychological distress to the surgeon. Further, the use of autologous bone graft from the patient's own iliac crests extends the operation and creates a second painful operative site. Because it is difficult to obtain a large enough quantity of autogenous bone with sufficient strength, homologous bank bone is generally used.

Interbody bone grafting involves the problems of strength and that of bone incorporation. Strong cortex bone (the outer layer) is required as a strut in the interbody position to prevent collapse of the disc space while healing occurs. The surgeon has the unfortunate requirement of having to fashion the required struts with handheld tools during the operation and these cortex bone struts are not wide enough for optimum load bearing and they anchor themselves by healing process that occurs very slowly over a matter of years. Further, soft cancellous bone, which heals more reliably over a matter of 12 to 18 months, is also required for a traditional interbody fusion.

It is well understood in orthopaedic surgery, that grafted bone heals by a process called "creeping substitution" in which blood capillaries first grow into the grafted bone, the grafted bone is reabsorbed, and then new bone cells are laid down along the bony matrix of the graft. During the time that the structural bone graft struts are being reabsorbed, motion must still be prevented in the involved segments and although a brace or cast is often used, the entire process has proven less reliable than desired. Homologous back bone, being more "foreign" requires a much longer time to grow together and has a higher failure rate estimated at three times the failure as with the patient's own bone. In effect, neither source of bone is optimum for the fusion procedure.

EP-A- 0042271 discloses an invertebral disc prosthesis comprising a body of biologically-acceptable material suitably dimensioned and shaped to replace a natural disc. In some embodiments a circular threaded hole is provided to secure a threaded end of a holding instrument to facilitate insertion of the prosthesis.

According to the present invention there is provided a prosthesis for insertion into transverse prepared grooves of substantially uniform transverse cross-section along the length thereof in opposed faces of adjoining vertebrae of a vertebral column, adjoining vertebrae having a disc space therebetween, said prosthesis, when inserted, comprising side-by-side rigid inert plugs of generally uniform transverse cross-section along the length thereof, to form transverse struts bottomed on the prepared grooves of adjoining vertebrae and maintaining a desired disc space between said adjoining vertebrae, each plug having an end face with tool receiving means extending internally of the plug for fixedly mounting the plug endwise on a tool to facilitate endwise insertion of the plug on the prepared grooves, a peripheral surface of the plugs being roughened over substantially the whole area thereof for interlocking with said prepared grooves to facilitate bone ingrowth from the vertebrae, each plug having at least one slot therethrough, adapted to receive bone graft material.

The plugs may have many different shapes and peripheral configureations.

The preferred plugs have one end thereof provided with an internally threaded axial hole and can have wings or radial slots radiating from this hole in the end face so that an insertion tool can be threaded into the hole and a sleeve of the insertion tool can be fitted into the slot recess. For example, a cylindrical plug, supported endwise on the tool can be inserted in prepared sites of the adjacent vertebrae and when properly positioned, the sleeve can be held against rotation and the tool unthreaded from the plug without shifting the plug. The radial slot is not needed where the plugs and sites are shaped to prevent rotation.

The sites are preferably formed by a drill surrounded by a drill guard with projecting teeth embedded in the posterior ends of adjacent vertebrae to correctly position the drill for forming channels in the opposed faces of adjacent vertebrae. The channels are sufficiently wide and long to include hard cortex bone but preferably do not extend completely through the anterior side of the vertebrae. Conversely, if the drill is inserted from the anterior site of the vertebrae, the drilling operation is stopped short of the posterior side.

The plugs can be made of an inert rigid metal, such as stainless steel, cobalt-chromium-molybdenum alloys, titanium or the like. The plugs can be provided with roughened surfaces forming pits, prongs, bristles, nubs, or the like irregularities for anchoring bone ingrowth. These roughened surfaces can be part of the plug body or a coating on the body, such as a resin polymer. Bristle or prong surfaces can be rigid or flexible and, if desired, shaped to facilitate insertion and resist retraction.

Preferred roughened surfaces include knurled surfaces, pitted surfaces, barbed surfaces, bristle surfaces and threaded surfaces engaging the prepared sites.

In one preferred embodiment the roughened surfaces are barbs or nubs radiating from the plug periphery or sides. The barbs may be deformable in the direction of insertion of the plug to facilitate insertion on the prepared site. The barbs and nubs can bite into the prepared sites upon attempted retraction of the plug.

The roughened surfaces can be on the periphery of a plastic material coating on the plug.

The plug is preferably composed of a radiolucent material.

The plug preferably has a length greater than its height and a width less than its height.

In one embodiment, the plug has at least one slot therethrough adapted to be packed with bone graft material.

Preferably the slotted plug has a longitudinal vertical slot and a transverse horizontal slot intersecting the vertical slot to receive the bone graft material.

In a preferred embodiment the plug has a myriad of radially extending nubs, the surface thereof separated by grooves. The nubs preferably have included front faces.

In one embodiment the plug has a bevelled front face and a rear face with a tool receiving recess.

A preferred threaded hole in the end face of the plug determines less than one half the length of the plug and has a diameter of less than 1/3 the diameter of the plug. Preferably, a wing or slot radiates diametrically from the hole in the end face of the plug, but terminates inwardly from the periphery of the plug.

A preferred polymer coating to form the roughened surface is nylon, a polyolefin, a vinyl, or the like resin resistant to deterioration in the environment of the implant.

The present invention now also provides rectangular vertebral prosthesis implant plugs or blocks fitting rectangular transverse or perpendicular channels or grooves cut in the adjoining faces of vertebral bodies having heights that will stretch the remaining annulus tissue of the discs therebetween still connecting the vertebrae. Flat-sided rectangular or square blocks or plugs are provided to fit these transverse rectangular channels or slots and have bevelled or tapered leading ends easily inserted into the open ends of the transverse slots to spread the vertebrae apart so that the top and bottom faces of each block or plug are tightly bottomed in the slot with the stretched disc tissues causing the vertebrae to grip the plugs. These plugs are inserted laterally or transversely of the vertebral column into the slots while mounted on the end of an insertion tool, have roughened surfaces to facilitate the bone ingrowth and can also have vertical or horizontal slots therethrough or intersecting vertical and horizontal slots, packed with bone graft material, such as stips of bone excised from the iliac crest of the pelvis. This implant material provides a block of living bone that grows all around and through the implant plug into the bone of the vertebrae.

Also, according to this invention, the blocks or plugs instead of being made of an inert metal, can be made of a radiolucent material, such as a plastic of the nylon, polycarbonate, polypropylene, polyacetal, polyethylene, and polysulfone type, preferably filled with glass or carbon fibres. These plastics can be injection molded, are light in weight, have great load carrying strength and provide improved X-ray visualisation of bone healing. Fiber reinforced plastics composed of such materials filled with glass or carbon fibers are also desirable. A preferred material is a polyether sulfone resin filled with glass and carbon fibers. Suitable carbon fiber composites are supplied under the tradename "VICTREX P.E.S." which is polyether sulfone filled with carbon fibers. Suitable graders are "4101 G.L.-30" which is a 30 percent fiber glass filled and "450 C.A.-30" which is a 30 percent carbon fiber filled. These materials are supplied from ICI Industries of Wilmington, Delaware. Carbon-carbon fiber plastics of the type sold by Fiber-Rite Corporation of Winona, Minnesota, are useful.

The invention will now be further described by way of examples with reference to the accompanying drawings, in which:

Fig.1 is a side elevational view of the lower portion of a human vertebral column with parts broken away and shown in section to illustrate prosthetic implants of this invention inserted between several of the lower vertebrae to support the vertebrae in place of the human disc therebetween which has been partially excised to remove damaged and herniated tissue,

Fig.2 is a posterior elevational view of a portion of Figure 1 taken along the line XIII-XIII of Figure 1.

Fig.3 is a transverse sectional view, with parts in elevation and broken away in section, along the line XIV-XIV of Figure 2.

Fig.4 is an enlarged fragmentary side elevational view, with parts broken away and shown in vertical section, illustrating the manner in which a trial or gauge plug or block of this invention is inserted in position in the transverse rectangular slots of adjoining vertebrae to stretch the remaining interposed disc tissue connected to these vertebrae and to gauge the sites for receiving a proper sized permanent implant.

Fig.5 is a plan view of a vertebrae disc with the interior pulp removed and with disc tissue partially excised to provide gaps or slots aligned with channels cut in the vertebrae to receive the plugs therethrough.

Fig.6 is a perspective view of a smooth faced trial or gauge plug or block for use as shown in Fig.4.

Fig.7 is a perspective view of a preferred form of permanent implant plug or block of this invention.

Fig.8 is a longitudinal vertical sectional view of the plug of Fig.7 taken along the line XIX-XIX of Fig.7.

Many other types of rough or irregular surfaces can be provided on the devices of this invention including porous metal coatings composed of metal balls and beads sintered on a rigid metal substrate as further disclosed in the aforesaid Patent No. 4,743,256.

The prosthetic implants are shown on the drawings as mounted in side-by-side parallel relation forming a pair of struts which maintain the disc space being snugly seated on hard cortex bone to carry the load. These implants have surfaces facilitating rapid bone ingrowth which will fuse the implants to the adjacent vertebrae in a relatively short growth period.

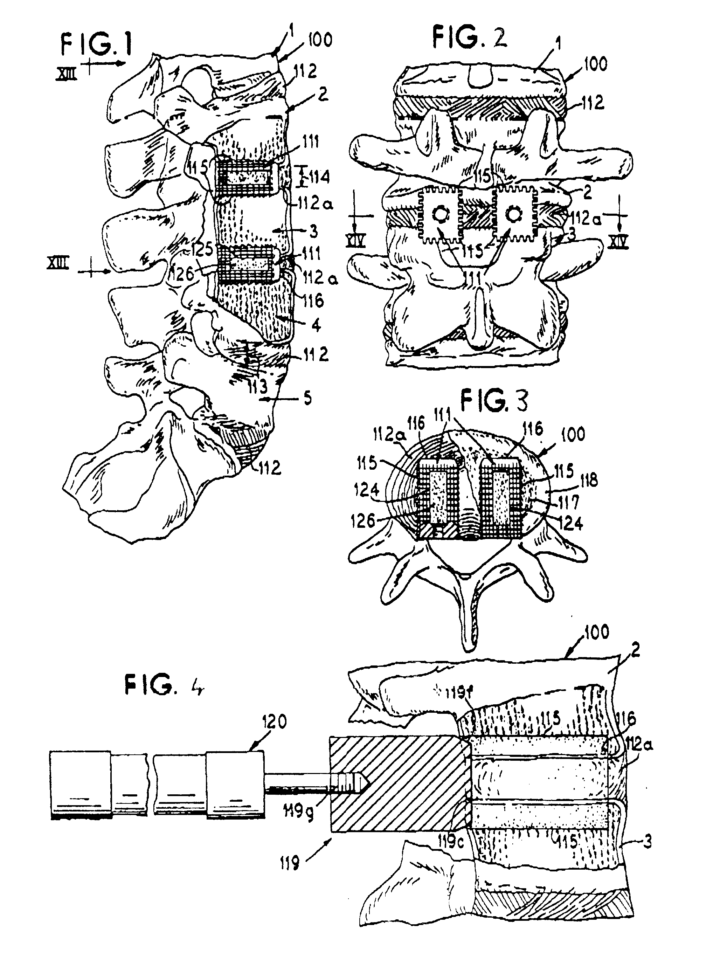

In Figures 1-3, the reference numeral 100 illustrates generally the lower portion of a human vertebral column with adjacent vertebrae supported on prosthetic implant blocks or plugs 111 of this invention.

Fig.4 shows the manner in which adjacent vertebrae are spread apart to stretch collapsed intervening disc tissue as a gauge or trial block of this invention is inserted laterally into transverse rectangular slots of adjoining vertebrae.

In FIGURE 1, the vertebral column 100 shows the five lower vertebrae Nos. 1-5. Adjacent vertebrae Nos. 2 and 3 and adjacent vertebrae Nos. 3 and 4 are separated by and supported on the prosthetic implant blocks or plugs 111 of this invention. Vertebrae Nos. 1 and 2 and vertebrae Nos. 4 and 5 are illustrated as supported on and separated by healthy or undamaged human discs 112 maintaining a normal disc space 113 between the adjoining vertebrae.

Damaged portions of the natural human discs 112 have been excised from the vertebrae Nos. 2 and 3 and Nos. 3 and 4 with the disc spaces 114 being maintained by the implant blocks or plugs 111. It is preferred to retain as much as possible of the healthy annulus tissue of the discs 112 between the vertebrae so that the remaining disc tissue 112a will at least partially surround the implants and will be held under tension by these implants. However, some of the remaining annulus disc tissue may have to be excised to open up spaces for the implant plugs 111.

The opposed faces of adjoining vertebrae have aligned flat-sided rectangular channels or grooves 115 cut therein transversely of the axis of column 100 to first snugly receive test blocks or plugs of this invention for determining the proper sizes for the permanent implants 111. These transverse channels 115 are sufficiently wide and deep to span the central soft cancellous bone and include the hard cortex bone of the adjacent vertebrae. The undamaged human disc tissue 112a remaining between the vertebrae is also cut or trimmed to receive the implants 111 so that as much healthy annulus fibrous tissue as is available will surround the implants.

The preferred flat-sided rectangular channels 115 have blind ends 116 to be abutted by the implants 111.

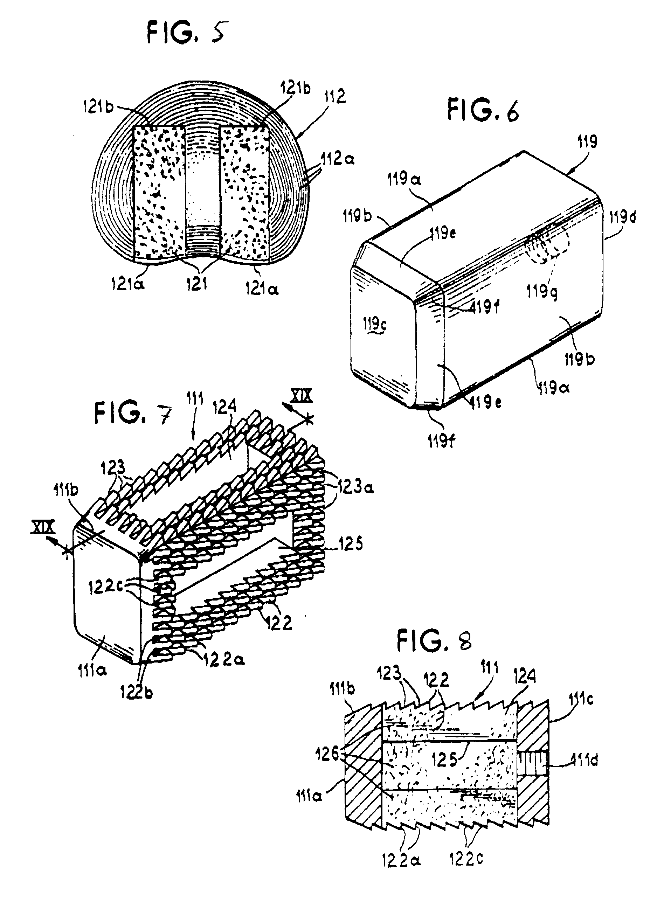

As shown in FIGURES 2 and 3, the implants 111 are in the form of a pair of side-by-side rectangular plugs inserted endwise into the transverse channels 115. These channels have flat bottoms and sidewalls to snugly embrace the top and bottom ends and side faces of the rectangular plugs. The soft cancellous bone of the vertebrae is illustrated at 117 in FIGURE 3 and is surrounded by the hard cortex bone 118. The channels 115 include portions of this hard cortex bone so that the implants 111 span the softer cancellous bone and rest on the hard cortex bone 118.

The channels 115 can be formed by a mortise cutting chisel tool and in the event disc tissue 112a blocks the paths for the plugs 111, tissue can be trimmed or spread apart to open up the paths.

The implant plugs of blocks 111, as shown in FIGURES 7 and 8, are rigid, inert, solid, flat-sided rectangles, higher than wide and longer than high. They are used in co-operation with trial or gauge blocks, such as 119, shown in FIGURE 6. These blocks 119 have flat, smooth sides and ends with flat top and bottoms 119a, flat sides 119b, a flat front end wall 119c, and a flat back end wall 119d. The front wall 119c is bevelled to a reduced rectangular nose surrounded by flat-sided tapered walls 119e with rounded corners 119f.

The back end wall 119d has an internally threaded blind axial hole 119g at the center of the wall.

The gauge blocks 119, in typical surgical operations, will have a length of about 25 mm, a width of about 11 mm and will vary in height from say, 13 to 17 mm, although it should be understood that these parameters may vary greatly and may depend on the size of the spinal column of the recipient. The tapers 119e are preferably about 30 degrees. The rounded corners 119f of the bevels eliminate sharp corners between the top, bottom and sides of the beveled faces.

As shown in FIGURE 4, a trial or gauge block 119 is selected for force-fitting into the channels 115 while mounted on a tool 120 threaded into the hole 119g. The beveled front end 119c of the block will pass through any portion of the disc tissue 112a covering the entrance mouths of the channels 115 by either cutting holes through the remaining tissue or by spreading apart the fibers of the disc to accept the gauge blocks 119.

As shown in FIGURE 5, the remaining healthy disc tissue 112a of a disc 112 between the channel cut vertebrae is trimmed to open up slots 121 permitting access of the gauge blocks 119 to the channels 115. These slots register with the channels 115 and can have open front ends 121a and blind back ends 121b. It is preferred to remove the nucleus pulposus from the damaged disc 112 leaving an annulus of fibrous tissue connecting the adjoining vertebrae and surrounding the inserted blocks.

A proper fitting gauge block 119 is selected by trial and error insertions into the channel cut vertebrae. These blocks are smooth faced and can be removed even when tightly fitted in the channels 115.

As shown in FIGURE 4, a gauge block 119, threaded on the end of on insertion tool 120 is selected to have a height greater than the height between the bottoms of opposed channels 115. Then, when this block is pushed through the open ends of the aligned channels 115, the beveled nose 119c will engage the bottoms of these channels forcing them apart as the block is pushed into the channels thereby stretching any disc tissue 112a still connecting the vertebrae. The block is pushed against the blind ends 116 of the channels and the tension on the disc fibers is determined. When a block 119 of sufficient size to properly load the disc tissue and to fit snugly in the channel, is located, a permanent implant plug 111 of a size just slightly greater than the gauge block is selected. Such a permanent plug is then threaded on the end of a tool 120, the gauge block 119 is withdrawn, and the permanent implant 111 on the tool is forced into a position in the channels 115.

A preferred permanent implant block or plug 111 is illustrated in FIGURES 7 and 8. This plug has about the same flat side dimensions as the selected gauge block, but has projected from these flat top, bottom and sidewalls, a pattern of raised annular nubs 122 providing a roughened surface, biting into and gripping the bottoms and sidewalls of the rectangular channels 115. These nubs are separated by annular grooves 123 and longitudinal channels 123a so that each nub 122 will have a flat vertical back wall 122a, a pair of flat vertical sidewalls 122b and an inclined front face 122c.

The plug 111 has the same reduced nose 111a surrounded by the same bevelled sidewalls 111b as the nose 119c and bevelled sidewall 119e of the gauge block 119. In addition a vertical back wall 111c is the same as the back wall 119d and contains the same internally threaded hole 111d as the back wall 119d of the gauge block 119.

Further, the implant plug 111 has a vertical slot 124 therethrough connecting the tops and bottoms of the plug. This vertical slot 124 is rectangular, has a width about 1/3 the width of the block and a length extending close to the front and rear ends of the plug.

This slot 124 is intersected centrally by a horizontal through slot 125. It will be understood that, alternately, the block 111 may have only a single horizontal or vertical slot.

The slots 124 and 125 provide cavities in the block or plug 11 which are filled with strips of bone implant 126 preferably harvested from the pelvis bone of the recipient. This bone material housed in the implant plugs 111 will soon grow out of the grooves or channels 124 and 125 into the radial and longitudinal channels between the nubs 122 surrounding the plugs 111 and will then grow into the bone tissue of the adjoining vertebrae.

The implant plug is easily inserted in the prepared sites 115 from the posterior side of the vertebrae by means of the tool assembly 120 having a threaded end 119c mating with a tapped hole 111d in an end face of the plug 111.

When the implant plug is pushed into its seated position between the vertebrae, the inclined front faces of the nubs 122 will accommodate the forward moving of the plug to the blind ends 116 of the channels 115, but the sharp apexes of the nubs will prevent retraction of the plugs since they will bite into the vertebrae bone. Therefore, once the plugs are seated in proper position, they will not shift from this position.

It is preferred that the height of the plugs 111 will be sufficient to maintain a tension load of about 9.1 to 13.6 kg (about 20 to 30 pounds) on the disc tissue. Such a tension load not only pulls the vertebrae tightly against the plugs, but also accelerates bone ingrowth.

The preferred prosthesis plugs or blocks 111 of this invention not only facilitate and simplify the surgical procedure but also accelerate interbody fusion of the vertebrae with the plug. The roughened surfaces provided by the nubs thus serve a multiple purpose of anchoring into the vertebrae, and providing channels for bone ingrowth.

## Claims

1. A prosthesis for insertion into transverse prepared grooves of substantially uniform transverse cross-section along the length thereof (115) in opposed faces of adjoining vertebrae (2-3, 3-4) of a vertebral column (100), adjoining vertebrae having a disc space therebetween, said prosthesis, when inserted, comprising side-by-side rigid inert plugs of generally uniform transverse cross-section along the length thereof (111) to form transverse struts bottomed on the prepared grooves of adjoining vertebrae and maintaining a desired disc space between said adjoining vertebrae, each plug having an end face with tool receiving means (111d) extending internally of the plug for fixedly mounting the plug endwise on a tool (120) to facilitate endwise insertion of the plug on the prepared grooves, a peripheral surface (122) of the plugs being roughened over substantially the whole area thereof for interlocking with said prepared grooves to facilitate bone ingrowth from the vertebrae, each plug having at least one slot (124,125) therethrough, adapted to receive bone graft material (126).

2. A prosthesis as claimed in claim 1, wherein the plug roughened surfaces are barbs (122) radiating from each plug and having leading faces (122c) sloping toward the tool receiving end of the plug to bite into the vertebrae and provide extended areas receiving bone ingrowth.

3. A prosthesis as claimed in claim 1 or 2, wherein the tool receiving means is an internally threaded hole (111d) in the end face of each plug for threaded engagement with the end of the tool.

4. A prosthesis as claimed in claim 3, wherein each plug comprises a radial slot in the end face radiating from the internally threaded hole.

5. A prosthesis as claimed in any one of the preceding claims, wherein each said plug ccomprises radiolucent material.

6. A prosthesis as claimed in any one of the preceding claims, wherein each plug comprises a nylon, polycarbonate, polypropylene, polyacetal or polysulfone plastic which is filled with glass or carbon fibres.

**Patentansprüche**

1. Prothese zum Einsetzen in querliegende vorbereitete Furchen mit im wesentlichen gleichförmig durchverlaufendem Querschnitt über ihre Länge (115) in gegenüberliegenden Flächen benachbarter Wirbel (2-3, 2-4) einer Wirbelsäule, wobei benachbarte Wirbel einen Scheibenabstand zwischen sich haben, wobei die Prothese, wenn sie eingefügt ist, nebeneinanderliegende starre inerte Pfosten mit im wesentlichen gleichförmig durchverlaufendem Querschnitt über ihre Länge (111) aufweisen, um Querstreben zu bilden, die auf den Boden der vorbereiteten Furchen aufgelegt sind und einen gewünschten Scheibenabstand zwischen den benachbarten Wirbeln aufrechterhalten, wobei jeder Pfosten eine Endfläche mit einer werkzeugaufnehmenden Einrichtung (111d) hat, die sich innen in dem Pfosten erstreckt, zum festen Anbringen des Pfostens am Ende auf einem Werkzeug (120) mit den Enden aneinander, um das Einfügen des Pfostens in die vorbereiteten Furchen mit den Enden aneinander zu erleichtern, wobei eine Umfangsfläche (122) der Pfosten über im wesentlichen deren gesamte Fläche zum Zusammenschluß mit den vorbereiteten Furchen aufgerauht ist, um das Einwachsen von Knochen vom Wirbel zu erleichtern, wobei jeder Pfosten wenigstens einen durchgehenden Schlitz (124, 125) hat, ausgelegt zum Aufnehmen von Knochentransplantatmaterial (126).

2. Prothese nach Anspruch 1, bei der die aufgerauhten Oberflächen des Pfostens Widerhaken (122) sind, die von jedem Pfosten strahlenartig ausgehen und Vorderflächen (122c) haben, die sich auf das werkzeugaufnehmende Ende des Pfostens zu abschrägen, um in den Wirbel zu schneiden und ausgedehnte Flächen, die einwachsenden Knochen aufnehmen, zu schaffen.

3. Prothese nach Anspruch 1 oder 2, bei der die werkzeugaufnehmende Einrichtung ein innen mit Gewinde versehenes Loch (111d) in der Endfläche jedes Pfostens zum Gewindeeingriff mit dem Ende des Werkzeuges ist.

4. Prothese nach Anspruch 3, bei der jeder Pfosten einen radialen Schlitz in der Endfläche aufweist, der von dem innen mit Gewinde versehenen Loch strahlenartig ausgeht.

5. Prothese nach einem der vorangehenden Ansprüche, bei der jeder der Pfosten strahlendurchlässiges Material aufweist.

6. Prothese nach einem der vorangehenden Ansprüche, bei der jeder Pfosten einen Nylon-, Polycarbonat-, Polypropylen-, Polyacetal- oder Polysulfonkunststoff aufweist, der mit Glas- oder Carbonfasern gefüllt ist.

**Revendications**

1. Prothèse à insérer dans des rainures transversales préparées ayant une section droite transversale sensiblement uniforme sur toute leur longueur (115) taillées dans les faces opposées de vertèbres adjacentes (2-3, 3-4) d'une colonne vertébrale (100), les vertèbres contiguës ayant entre elles un espace discal, ladite prothèse, une fois mise en place, comprenant des fiches inertes rigides placées côte à côte ayant une section droite transversale généralement uniforme sur leur longueur (111) pour former des jambes de force transversales enfoncées sur les rainures préparées des vertèbres voisines et en maintenant un espace discal souhaité entre lesdites vertèbres voisines, chaque fiche ayant une face d'extrémité comportant un moyen de réception d'outil (111d) orienté à l'intérieur de la fiche de manière à monter rigidement la fiche en bout sur un outil (120) afin de faciliter l'insertion en bout de la fiche sur les rainures préparées, une surface périphérique (122) des fiches étant rendue rugueuse sensiblement sur toute sa surface de façon à assurer un verrouillage avec lesdites rainures préparées et à faciliter la croissance de l'os à partir des vertèbres, chaque fiche présentant au moins une fente (124,125) qui la traverse, prévue pour recevoir un greffon osseux (126).

2. Prothèse selon la revendication 1, dans laquelle les surfaces rugueuses de la fiche comprennent des barbelures (122) partant de chaque fiche et ayant des faces avant (122c) en pente vers l'extrémité de réception d'outil de la fiche afin de se fixer dans les vertèbres et de créer des zones étendues dans lesquelles la croissance osseuse peut s'effectuer.

3. Prothèse selon la revendication 1 ou 2, dans laquelle le moyen de réception de l'outil est un orifice fileté intérieurement (111d) formé dans la face d'extrémité de chaque fiche pour permettre une prise par vissage de l'extrémité de l'outil.

4.   Prothèse selon la revendication 3, dans laquelle chaque fiche comporte une fente radiale dans la face d'extrémité partant de l'orifice fileté intérieurement.

5.   Prothèse selon l'une quelconque des revendications précédentes, dans laquelle chacune desdites fiches est constituée d'un matériau radiotransparent.

6.   Prothèse selon l'une quelconque des revendications précédentes, dans laquelle chaque fiche est constituée d'une matière plastique en nylon, polycarbonate, polypropylène, polyacétate ou polysulfone, chargé de fibres de verre ou de fibres carbone.

FIG.1

FIG. 2

FIG. 3

FIG. 4

FIG. 5

FIG. 6

FIG. 7

FIG. 8